# EUROPÄISCHE PATENTSCHRIFT

(11) **EP 3 265 169 B1**
(45) Veröffentlichungstag und Bekanntmachung des Hinweises auf die Patenterteilung: **10.06.2020**
(21) Anmeldenummer: 16706564.8
(22) Anmeldetag: 17.02.2016
(51) Int. Cl.: A61M 39/10, A61M 39/16, A61M 39/22, F16K 11/00, F16K 11/085

(54) **MEDIZINISCHE FLUIDSTEUERVORRICHTUNG UND PARTIKELFILTER HIERFÜR**
MEDICAL FLUID CONTROL DEVICE AND A PARTICULATE FILTER FOR SAME
DISPOSITIF MÉDICAL DE COMMANDE D'ÉCOULEMENT DE FLUIDE ET FILTRE ANTI-PARTICULES POUR CE DISPOSITIF

(30) Priorität: 04.03.2015 DE 102015203863
(43) Veröffentlichungstag der Anmeldung: 10.01.2018
(73) Patentinhaber: B. Braun Melsungen AG, 34212 Melsungen (DE)
(72) Erfinder: KUNSCHAK, Ralf, 6130 Willisau (CH)
(74) Vertreter: Patentanwälte Ruff, Wilhelm, Beier, Dauster & Partner mbB
(86) Internationale Anmeldenummer: PCT/EP2016/053387
(87) Internationale Veröffentlichungsnummer: WO 2016/139064

(56) Entgegenhaltungen:
- WO-A1-2011/024725
- WO-A1-2014/170379
- WO-A2-2006/025054
- CN-U- 202 289 023
- KR-Y1- 200 475 695
- US-A- 4 581 014
- US-A1- 2010 022 968
- US-A1- 2012 067 429

## Beschreibung

Die Erfindung betrifft eine medizinische Fluidsteuervorrichtung für ein medizinisches Fluidleitungssystem mit einem Fluidströmungsgehäuse, das wenigstens einen Anschlussport für ein Anschließen und Entfernen einer externen medizinischen Fluidführungskomponente sowie wenigstens einen Verbindungsport für eine Verbindung zu einer weiteren Funktionskomponente des medizinischen Fluidleitungssystems aufweist, sowie mit einem Stellorgan zur Steuerung einer Fluidströmung relativ zu dem wenigstens einen Anschlussport und/oder dem wenigstens einen Verbindungsport sowie einen Partikelfilter hierfür.

Eine medizinische Fluidsteuervorrichtung in Form eines Dreiwegehahns für ein medizinisches Infusionssystem ist allgemein bekannt. Eine derartige Fluidsteuervorrichtung ist vorgesehen, um einer Patientenleitung eines medizinischen Fluidleitungssystems Medikamente zuführen zu können. Entsprechende Arzneimittel können in Fluidspeichern gespeichert sein, die aus Glas bestehen oder Gummiverschlussstopfen aufweisen. Die zu verabreichenden Medikamente können demzufolge mit Glas- oder Gummipartikeln oder anderen Verunreinigungen, verursacht bei der Produktion oder der Verpackung oder der Vorbereitung zur Anwendung wie Durchstechen des Stopfens oder Aufbrechen der Glas-Vials, versehen sein. Die Verunreinigung derartiger zu verabreichender Medikamente mit Glaspartikeln oder Gummipartikeln ist bekannt. Um zu vermeiden, dass derartige Verunreinigungen dem Patienten zugeführt werden, ist es vorgesehen, bei der Zugabe derartiger Medikamente in eine entsprechende Fluidleitung zwischen dem mit den Medikamenten versehenen Fluidspeicher und der Patientenleitung Filterbauteile in-line einzufügen. Es kann jedoch passieren, dass medizinisches Pflegepersonal im klinischen Alltagsbetrieb die Zwischenfügung entsprechender Filterbauteile zwischen Fluidspeicher und Patientenleitung vergisst.

Aufgabe der Erfindung ist es, eine medizinische Fluidsteuervorrichtung und einen Partikelfilter der eingangs genannten Art zu schaffen, die sicherstellt, dass eine unerwünschte Zugabe von Fremdpartikeln bei Anschluss einer medizinischen Fluidführungskomponente, insbesondere eines Fluidspeichers, vermieden wird.

Diese Aufgabe wird für die Fluidsteuervorrichtung dadurch gelöst, dass in dem wenigstens einen Anschlussport und/oder dem wenigstens einen Verbindungsport jeweils ein Partikelfilter mit einer Porenweite zwischen 2 µm und 15µm integriert ist. Vorzugsweise weist der Partikelfilter eine Porenweite von 5 µm oder größer auf. Durch die Integration des Partikelfilters in den Anschlussport und/oder den Verbindungsport werden Fremdpartikel, die gegebenenfalls über eine externe medizinische Fluidführungskomponente zugeführt werden, im Anschlussport zurückgehalten. Eine Zugabe in das medizinische Fluidleitungssystem wird zuverlässig vermieden. Eine Verunreinigung einer Patientenleitung innerhalb des Fluidleitungssystems durch entsprechende Fremdpartikel wird demzufolge erfindungsgemäß ebenfalls vermieden. Die erfindungsgemäße Lösung eignet sich in besonders vorteilhafter Weise für den Einsatz bei einem medizinischen Infusionssystem, das eine Patientenleitung umfasst und bei dem die Fluidsteuervorrichtung als Mehrwegehahn, vorzugsweise als Dreiwegehahn, ausgeführt ist. Ein Fluidströmungsgehäuse der medizinischen Fluidsteuervorrichtung gemäß der Erfindung ist vorzugsweise aus einem geeigneten Kunststoff, insbesondere aus Polyamid oder Polycarbonat, hergestellt. Für die Verabreichung von Zytostatika wird ein Fluidströmungsgehäuse aus Polyamid bevorzugt, da Polyamid eine ausreichende Spannungsresistenz aufweist. Als Fluidführungskomponente ist bei einem derartigen medizinischen Infusionssystem vorzugsweis eine Spritze vorgesehen.

In Ausgestaltung der Erfindung ist der Partikelfilter kraft- und/oder form- und/oder stoffschlüssig in dem Anschlussport oder dem Verbindungsport gehalten. Vorzugsweise ist der Partikelfilter durch Einpressen (Kraftschluss), durch Einkleben (Stoffschluss) oder Einschweißen (vorzugsweise Ultraschallschweißen) (Stoffschluss) oder auch durch eine vorzugsweise unlösbare Rastverbindung (Formschluss) in dem Anschlussport oder dem Verbindungsport befestigt.

Gemäß der Erfindung ist der Partikelfilter als räumlich innerhalb des Anschlussports oder des Verbindungsports längserstreckter Hutkörper gestaltet. Die Gestaltung als Hutkörper gewährleistet eine große nutzbare Filterfläche, so dass ein Verstopfen des Anschlussports zuverlässig vermieden werden kann.

Weiterhin gemäß der Erfindung ist der Hutkörper als einseitig offener Hohlkörper mit einer sich verjüngenden Gestaltung ausgeführt. Bei einer sich verjüngenden Gestaltung des Hutkörpers ist die Filterfläche entweder als sich stetig verjüngende oder als sich abschnittsweise verjüngende Gestaltung ausgeführt, insbesondere als konisch zulaufende Filterfläche. Es ist auch möglich, eine hohlzylindrische mit einer konisch zulaufenden Filterfläche zu kombinieren. Die entsprechende Filterfläche wird vorzugsweise durch wenigstens einen Trägerring gestützt, der gegenüber dem Filtermaterial der Filterfläche formstabil ausgeführt ist und neben der Halterung des Filtermaterials auch zur Abdichtung des Partikelfilters innerhalb des Anschlussports oder des Verbindungsports dient.

Weiterhin gemäß der Erfindung weist der Hutkörper eine schmal zulaufende Spitze auf. Je nach Ausrichtung des Hutkörpers ist hierdurch eine Konzentration von aufgefangenen Fremdpartikeln im Bereich der Spitze oder außenseitig im Bereich eines der Spitze gegenüberliegenden Rands des Hutkörpers erzielbar.

In weiterer Ausgestaltung der Erfindung weist der Hutkörper an einem Stirnendbereich einen Trägerring auf, der an einer Innenwandung des Anschlussports oder der Verbindungsports befestigt ist. Der Trägerring ist vorzugsweise - wie das Fluidströmungsgehäuse - aus einem geeigneten Kunststoff, insbesondere aus Polyamid oder Polycarbonat, hergestellt, je nach Einsatzzweck. Für die Verabreichung von Zytostatika über den Anschlussport oder dem Verbindungsport ist der Trägerring vorteilhaft aus Polyamid hergestellt.

In weiterer Ausgestaltung der Erfindung ist der Partikelfilter in dem Anschlussport oder dem Verbindungsport derart befestigt, dass die Spitze des Hutkörpers distal oder proximal zu einem Anschlussbereich des Anschlussports oder des Verbindungsports ausgerichtet ist. Vorzugsweise ist der Anschlussport oder der Verbindungsport mit einem Luer-Lock-Anschluss versehen. Die Spitze des Hutkörpers kann - je nach Variante - demzufolge ins Innere des Fluidströmungsgehäuses hinein oder in Richtung des außenliegenden Anschlussbereichs des Anschlussports oder des Verbindungsports nach außen gerichtet sein.

In weiterer Ausgestaltung der Erfindung umfasst der Partikelfilter zwei axial zueinander beabstandete Trägerringe, zwischen denen ein Haltebereich für eine Filterfläche erstreckt ist. In vorteilhafter Weise erstrecken der Haltebereich und die Filterfläche sich in einer zwischen den Trägerringen verlaufenden, schrägen Ebene. In besonders vorteilhafter Weise weisen die zueinander beabstandeten Trägerringe unterschiedliche Außendurchmesser auf. Die unterschiedlichen Außendurchmesser ermöglichen eine Positionierung des Partikelfilters in einem sich konisch verjüngenden oder konisch erweiternden Kanal eines entsprechenden Anschlussports oder Verbindungsports. Der mit den beiden Trägerringen und dem dazwischenliegenden Haltebereich versehene Partikelfilter weist eine hohe Stabilität auf. Wenigstens einer der beiden Trägerringe kann innerhalb eines entsprechenden Anschlussports oder Verbindungsports an einer Ringschulter eines entsprechenden Strömungskanals des Verbindungsports oder des Anschlussports abgestützt sein.

In weiterer Ausgestaltung der Erfindung ist die Innenwandung des Anschlussports oder des Verbindungsports mit einer Ringprofilierung versehen, die komplementär auf eine Ringaußenkontur des Trägerrings abgestimmt ist. Die Ringprofilierung und die Ringaußenkontur sind derart aufeinander abgestimmt, dass sich für den Trägerring ein flächenbündiger Sitz in dem Anschlussport oder dem Verbindungsport ergibt, um eine sichere Abdichtung zwischen dem Partikelfilter und der Innenwandung des Anschlussports oder des Verbindungsports zu erzielen.

In weiterer Ausgestaltung der Erfindung ist die Ringprofilierung als Ringschulter oder als Ringwandung mit einem sich stetig ändernden Durchmesser, insbesondere als konisch gestaltete Ringwandung, ausgeführt. Eine Ringwandung mit einem sich stetig ändernden Durchmesser wird vorzugsweise durch eine sich konisch verjüngende oder eine sich konisch erweiternde ringförmige Wandung gebildet. Als Ringstufe ist eine Ringschulter im Bereich der Innenwandung des Anschlussports oder des Verbindungsports vorgesehen.

Für den Partikelfilter wird die der Erfindung zugrundeliegende Aufgabe dadurch gelöst, dass der Partikelfilter die den Partikelfilter betreffenden Merkmale von wenigstens einer der zuvor beschriebenen Ausgestaltungen aufweist.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in perspektivischer Explosionsdarstellung eine Ausführungsform einer erfindungsgemäßen medizinischen Fluidsteuervorrichtung,
- Fig. 2: eine Seitenansicht der Fluidsteuervorrichtung nach Fig. 1,
- Fig. 3: einen Schnitt durch die Fluidsteuervorrichtung nach Fig. 2 entlang der Schnittlinie III-III in Fig. 2,
- Fig. 4: eine weitere Schnittdarstellung der Fluidsteuervorrichtung nach Fig. 2 entlang der Schnittlinie IV-IV in Fig. 2,
- Fig. 5: in vergrößerter Darstellung einen Ausschnitt der Darstellung nach Fig. 4,
- Fig. 6: in einer Seitenansicht eine weitere Ausführungsform einer erfindungsgemäßen Fluidsteuervorrichtung ähnlich Fig. 2,
- Fig. 7: einen Schnitt durch die Fluidsteuervorrichtung nach Fig. 6 längs der Schnittlinie VII-VII in Fig. 6,
- Fig. 8: eine Schnittdarstellung der Fluidsteuervorrichtung nach Fig. 6 entlang der Schnittlinie VIII-VIII in Fig. 6,
- Fig. 9: einen vergrößerten Ausschnitt der Schnittdarstellung nach Fig. 8,
- Fig. 10: eine weitere Ausführungsform einer erfindungsgemäßen Fluidsteuervorrichtung in einer Seitenansicht,
- Fig. 11: einen Schnitt durch die Fluidsteuervorrichtung nach Fig. 10 entlang der Schnittlinie XI-XI in Fig. 10,
- Fig. 12: eine weitere Schnittdarstellung der Fluidsteuervorrichtung nach Fig. 10 entlang der Schnittlinie XII-XII in Fig. 10,
- Fig. 13: einen vergrößerten Ausschnitt der Schnittdarstellung nach Fig. 12,
- Fig. 14: in perspektivischer Explosionsdarstellung eine weitere Ausführungsform einer erfindungsgemäßen medizinischen Fluidsteuervorrichtung,
- Fig. 15: eine Schnittdarstellung eines Teilbereichs der Fluidsteuervorrichtung nach Fig. 14 mit integriertem Partikelfilter und
- Fig. 16: in perspektivischer Explosionsdarstellung den in die Fluidsteuervorrichtung gemäß den Fig. 14 und 15 integrierten Partikelfilter.

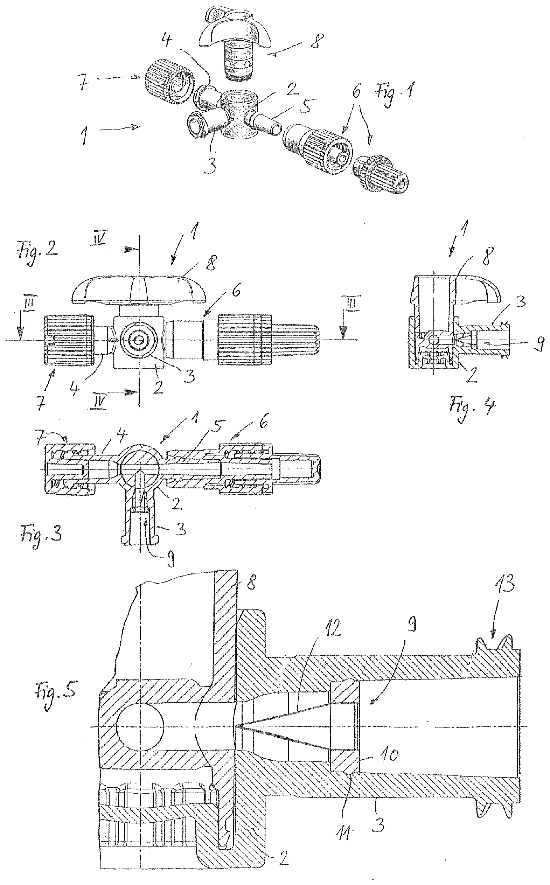

Eine medizinische Fluidsteuervorrichtung, wie sie anhand der verschiedenen Ausführungsformen gemäß den Fig. 1 bis 13 dargestellt ist, ist als Dreiwegehahn für ein medizinisches Infusionssystem für die Verabreichung von Arzneimitteln, insbesondere für die Verabreichung von Zytostatika, vorgesehen. Der Dreiwegehahn 1, 1a, 1b gemäß den Fig. 1 bis 13 ist jeweils mit einem Fluidströmungsgehäuse 2, 2a, 2b versehen, an dem drei zu unterschiedlichen Richtungen hin abragende Porte 3, 4, 5; 3a, 4a, 5a; 3b, 4b, 5b angeordnet sind. In dem Fluidströmungsgehäuse 2, 2a, 2b ist ein Stellorgan 8, 8a, 8b um eine Hochachse des Fluidströmungsgehäuses 2, 2a, 2b drehbeweglich gelagert. Das Stellorgan 8, 8a, 8b wird auch als Küken bezeichnet. In dem Stellorgan 8, 8a, 8b sind nicht näher bezeichnete Fluidleitungskanäle vorgesehen, die eine Fluidströmung zwischen den drei Porten 3, 4, 5; 3a, 4a, 5a; 3b, 4b, 5b je nach Stellung des Stellorgans 8 freigeben oder sperren. Der Port 3, 3a, 3b stellt einen Anschlussport im Sinne der Erfindung dar. Der entsprechende Anschlussport 3, 3a, 3b wird anhand der Zeichnungen nachfolgend näher beschrieben. Die beiden Porte 4, 5; 4a, 5a; 4b, 5b stellen Verbindungsporte im Sinne der Erfindung dar. Der Verbindungsport 4 ist mit einem Luer-Lock-Anschluss versehen und dient dazu, eine weitere Funktionskomponente des Fluidleitungssystems, insbesondere einen Fluidspeicher in Form eines Beutels mit dem zu verabreichenden Medium (Flüssigkeit), anzuschließen. Der Verbindungsport 5 bildet eine Verbindung zu einer Patientenleitung des Infusionssystems, die zu einem in Behandlung befindlichen Patienten führt. Die Patientenleitung wird über entsprechende Anschlusselemente 6 mit dem Verbindungsport 5 verbunden. Dabei wird eine äußere Abdeckkappe der Anschlusselemente 6 entfernt. Ein auf dem Verbindungsport 5 gesichertes Anschlusselement 6 ist mit einem männlichen Luer-Lock-Anschluss versehen, mit dem ein komplementäres Luer-Lock-Anschlussteil der Patientenleitung verbunden werden kann. Der diametral gegenüberliegende Verbindungsport 4 ist ebenfalls mit einer Verschlusskappe 7 versehen, die entfernt wird, sobald der Verbindungsport 4 benutzt wird.

In jedem Anschlussport 3, 3a, 3b der verschiedenen Dreiwegehähne 1, 1a, 1b gemäß den Fig. 1 bis 13 ist jeweils ein Partikelfilter 9, 9a, 9b integriert. Die Integration des jeweiligen Partikelfilters 9, 9a, 9b ist bei den drei Ausführungsformen unterschiedlich gestaltet. Nachfolgend wird anhand der Zeichnungen auf die Unterschiede eingegangen. Bei allen Ausführungsformen bildet der jeweilige Partikelfilter 9, 9a, 9b einen Hutkörper 12, 12a, 12b, der ein dreidimensionales Formteil darstellt, das längs einer Längserstreckung des Anschlussports 3 ausgerichtet ist. Bei allen Partikelfiltern 9, 9a, 9b ist der Hutkörper 12, 12a, 12b spitz zulaufend gestaltet. Jeder Hutkörper 12, 12a, 12b ist aus einem geeigneten Filtermaterial hergestellt mit einer Porenweite zwischen 2 µm und 15 µm gestaltet ist. Jeder Hutkörper 12, 12a, 12b ist in jeweils einem Trägerring 10, 10a, 10b gehalten. Bei den dargestellten Ausführungsbeispielen ist der jeweilige Hutkörper 12, 12a, 12b in den jeweiligen Trägerring 10, 10a, 10b eingeklebt. Der jeweilige Trägerring 10, 10a, 10b ist - wie das Fluidströmungsgehäuse 2, 2a, 2b - aus Polyamid hergestellt.

In gleicher Weise kann im Verbindungsport 4, 4a, 4b ein entsprechender Partikelfilter integriert sein.

Bei der Ausführungsform nach den Fig. 1 bis 5 ist der Partikelfilter 9 in dem Anschlussport 3 formschlüssig gehalten. Hierzu ist im Bereich einer Innenwandung des Anschlussports 3 zum einen eine Ringschulter vorgesehen, auf der der Trägerring 10 des Partikelfilters 9 axial abgestützt ist. Zudem ist die Innenwandung im Bereich des Trägerrings 10 mit einer umlaufenden, rinnenförmigen Ringnut versehen, in die eine komplementär gestaltete Ringprofilierung im Bereich eines Außenrands des Trägerrings 10 eingerastet ist. Die Ringschulter und die Ringnut im Bereich der Innenwandung des Anschlussports 3 stellen Ringprofilierungen im Sinne der Erfindung dar und dienen zur Abdichtung des Trägerrings 3 relativ zur Innenwandung des Anschlussports 3. Eine Ringaußenkontur des Trägerrings 10 ist entsprechend flächenbündig auf die Innenwandung des Anschlussports 3 im Bereich der Ringprofilierung abgestimmt.

Die Ringschulter der Innenwandung des Anschlussports 3 ist derart axial in Abstand zu dem Stellorgan 8 positioniert, dass eine Spitze des Hutkörpers 12 des Partikelfilters 9, die koaxial zu einer Mittellängsachse des Anschlussports 3 nach innen in Richtung zum Stellorgan 8 gerichtet ist, nicht in eine Drehbewegungsbahn des Stellorgans 8 hineinragt. Die Spitze des Hutkörpers 12 endet unmittelbar vor einer Außenkontur des Stellorgans 8. Der Anschlussport 3 ist mit einem Anschlussbereich 13 an seinem relativ zum Stellorgan 8 distalen Stirnendbereich versehen, der als weiblicher Luer-Lock-Anschluss gestaltet ist.

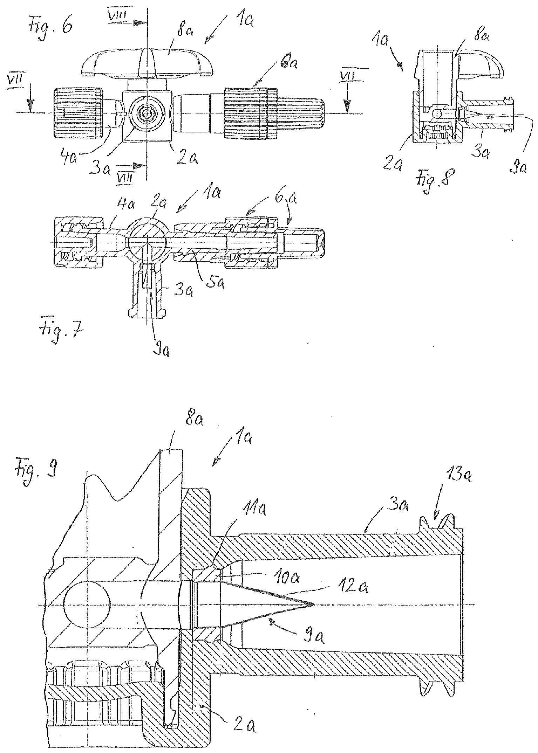

Bei der Ausführungsform nach den Fig. 6 bis 9 weist der Dreiwegehahn 1a einen in dem Anschlussport 3a integrierten Partikelfilter 9a auf, der einen zu dem Anschlussbereich 13a nach außen gerichteten Hutkörper 12a umfasst. Bei dieser Ausführungsform ist eine Ringprofilierung 11a im Bereich der Innenwandung des Anschlussports 3a gegenüber der Ausführungsform nach Fig. 5 proximal zum Stellorgan 8a nach innen versetzt positioniert. Die Ringprofilierung 11a ist in gleicher Weise als Ringnut in der Innenwandung ausgeführt, wie dies bei der Ausführungsform gemäß Fig. 5 der Fall ist. Sie dient demzufolge in gleicher Weise auch zur Abdichtung. Auch der Trägerring 10a weist eine komplementäre Ringaußenkontur auf, die ein Verrasten des Trägerrings 10a in der Ringprofilierung 11a der Innenwandung des Anschlussports 3a gewährleistet. Der Hutkörper 12a ragt vom Trägerring 10a aus längserstreckt distal zum Anschlussbereich 13a hin ab. Der Partikelfilter 9a ist demzufolge entgegengesetzt zu dem Partikelfilter 9 im Anschlussport 3a ausgerichtet.

Anhand der Fig. 3 und 7 ist erkennbar, dass die Spitze des jeweiligen Hutkörpers 12, 12a nicht kegelförmig, sondern vielmehr keilförmig zulaufend gestaltet ist, so dass die Spitze eine quer zur Längsrichtung des Anschlussports 3a ausgerichtete Kante definiert.

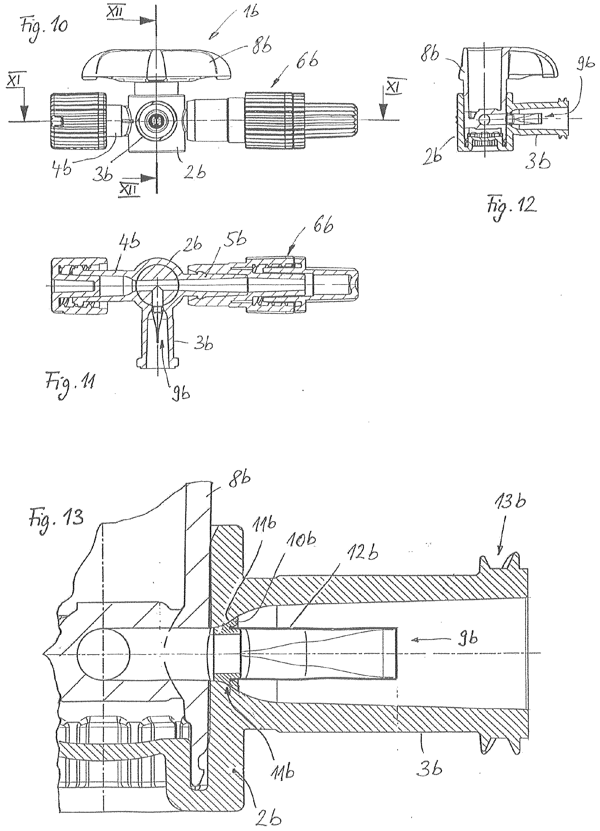

Bei der Ausführungsform nach den Fig. 10 bis 13 ist der Partikelfilter 9b stoffschlüssig durch Verschweißung oder Verklebung in dem Anschlussport 3b integriert. Hierzu ist eine Ringwandung 11b der Innenwandung des Anschlussports 3b im Querschnitt stetig verjüngt in Richtung des Stellorgans 8b gestaltet. Der Trägerring 10b weist eine komplementäre Ringaußenkontur auf, die einen flächenbündigen Sitz des Trägerrings 10b im Bereich der Ringwandung 11b gewährleistet. Die Befestigung des Trägerrings 10b an der Ringwandung 11b erfolgt entweder durch Ultraschallverschweißung oder durch Verklebung mittels eines geeigneten Klebstoffs. Der Hutkörper 12b ist axial mit seinem der Spitze gegenüberliegenden Stirnrand in eine axiale Ringnut des Trägerrings 10b eingesteckt und in dieser ebenfalls durch Verklebung oder Verschweißung stoffschlüssig gehalten. Sowohl die Ringwandung 11b als auch der Trägerring 10b sind in eingefügtem Zustand des Partikelfilters 9b an einem dem Stellorgan 8b zugewandten Endbereich des Anschlussports 3b positioniert, wohingegen der Hutkörper 12b axial nach außen in Richtung des Anschlussbereichs 13b abragt. Auch der Anschlussbereich 13b, der einen relativ zum Stellorgan 8b distalen Stirnendbereich des Anschlussports 3b bildet, ist mit einem männlichen Luer-Lock-Anschluss versehen.

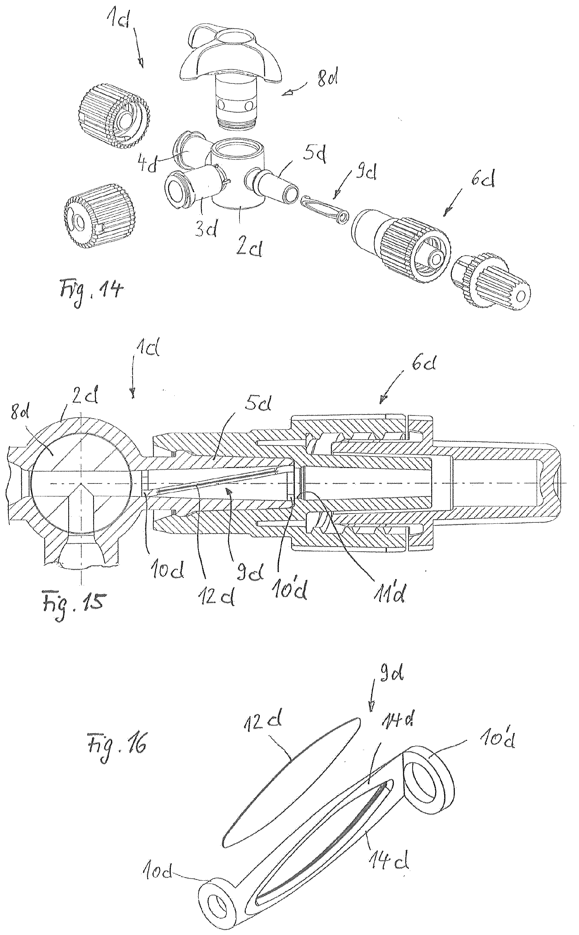

Die nicht beanspruchte medizinische Fluidsteuervorrichtung nach den Fig. 14 bis 16 ist ebenfalls als Dreiwegehahn 1d ausgeführt und entspricht im Wesentlichen den zuvor beschriebenen Ausführungsformen. Baugleiche oder funktionsgleiche Abschnitte und Teile des Dreiwegehahns 1d sind mit gleichen Bezugszeichen unter Hinzufügung des Buchstabens d versehen. Zur Vermeidung von Wiederholungen wird bezüglich der baugleichen und funktionsgleichen Teile und Abschnitte des Dreiwegehahns 1d auf die Offenbarung der zuvor beschriebenen Ausführungsformen verwiesen. Nachfolgend wird auf die Unterschiede des Dreiwegehahns 1d zu den zuvor beschriebenen Ausführungsformen eingegangen.

Wesentlicher Unterschied bei der Ausführungsform nach den Fig. 14 bis 16 ist es, dass bei dem Dreiwegehahn 1d in dem Verbindungsport 5d ein Partikelfilter 9d integriert ist. Der Verbindungsport 5d weist einen sich von dem Stellorgan 8d ausgehend zu einem äußeren Stirnendbereich konisch erweiternden Strömungskanal auf. Auf den Verbindungsport 5d ist das Anschlusselement 6d aufgerastet, das sich mittels einer Ringschulter 11'd axial auf dem Stirnendbereich des Verbindungsports 5d abstützt. In dem Strömungskanal des Verbindungsports 5d ist der Partikelfilter 9d integriert. Der Partikelfilter 9d wird gebildet durch zwei parallel zueinander axial beabstandete Trägerringe 10d und 10'd, die unterschiedliche Außendurchmesser aufweisen. Zudem ist eine Umfangskontur des Trägerrings 10d komplementär zu der Innenkontur des Strömungskanals des Verbindungsports 5d konisch gestaltet. Der Außendurchmesser des Trägerrings 10d ist kleiner als der Außendurchmesser des axial beabstandeten Trägerrings 10'd. Der Trägerring 10d und der Trägerring 10'd sind über einen Haltebereich 14d einstückig miteinander verbunden, der eine längliche Aufnahmeöffnung umgibt. In der hier dargestellten länglichen Aufnahmeöffnung ist ein Filtermaterial 12d gehalten, das gemäß Fig. 19 eine komplementär längliche Außenkontur aufweist. Sowohl der Haltebereich 14d als auch das Filtermaterial 12d erstrecken sich zumindest im Wesentlichen längs einer gemeinsamen Ebene, die zwischen dem Trägerring 10d und dem Trägerring 10'd schräg verläuft. Das Filtermaterial 12d ist in dem Haltebereich 14d fest eingespannt. Zudem umgibt der Haltebereich 14d das Filtermaterial 12d dicht. Die beiden Trägerringe 10d und 10'd sind mit unterschiedlich großen Innendurchmessern versehen, durch die hindurch entsprechendes Fluid innerhalb des Strömungskanals des Verbindungsports 5d im Betrieb des Dreiwegehahns 1d strömt, wobei entsprechende Partikel durch das Filtermaterial 12d des Partikelfilters 9d zurückgehalten werden. Der Trägerring 10'd stützt sich axial gegen die Ringschulter 11'd des Anschlusselements 6d ab. Der Partikelfilter 9d wird bei seiner Montage demzufolge in einfacher Weise mit dem kleineren Trägerring 10d voraus axial in das Stirnende des Verbindungsports 5d eingeführt, bis ein Außenrand des Trägerrings 10d kraftschlüssig und dicht an der Innenkontur des Strömungskanals des Verbindungsports 5d unmittelbar benachbart zu dem Stellorgan 8d geklemmt ist. Die Abmessungen der Trägerringe 10d und 10'd sind derart abgestimmt, dass der größere Trägerring 10'd sich in der Klemmposition des Trägerrings 10d zumindest weitgehend bündig mit der Stirnseite des Verbindungsports 5d befindet. Sobald nun das Anschlusselement 6d axial aufgerastet wird, sichert die Ringschulter 11'd den Trägerring 10'd axial in dem Verbindungsport 5d. Falls der Trägerring 10'd vor dem Aufrasten des Anschlusselements 6d noch geringfügig axial über die Stirnseite des Verbindungsports 5d hinausragt, drückt die Ringschulter 11'd den Trägerring 10'd zusätzlich um einen geringen Betrag axial in den Verbindungsport 5d hinein, wodurch eine zusätzliche Klemmspannung auf den Partikelfilter 9d aufgebracht wird, die die Befestigung des Partikelfilters 9d sowie die Abdichtung innerhalb des Strömungskanals des Verbindungsports 5d weiter verbessert.

## Patentansprüche

1. Medizinische Fluidsteuervorrichtung für ein medizinisches Fluidleitungssystem mit einem Fluidströmungsgehäuse (2-2b), das wenigstens einen Anschlussport (3-3b) für ein Anschließen und Entfernen einer externen medizinischen Fluidführungskomponente sowie wenigstens einen Verbindungsport (4-4b, 5-5b) für eine Verbindung zu einer weiteren Funktionskomponente des medizinischen Fluidleitungssystems aufweist, sowie mit einem Stellorgan (8-8b) zur Steuerung einer Fluidströmung relativ zu dem wenigstens einen Anschlussport (3-3b) und/oder dem wenigstens einen Verbindungsport (4-4b, 5-5b), wobei in dem wenigstens einen Anschlussport (3-3b) und/oder dem wenigstens einen Verbindungsport (4-4b, 5-5b) jeweils ein Partikelfilter (9-9b) mit einer Porenweite zwischen 2 µm und 15 µm integriert ist, wobei der Partikelfilter (9-9b) als räumlich innerhalb des Anschlussports (3-3b) oder des Verbindungsports längserstreckter Hutkörper (12-12b) gestaltet ist, und wobei der Hutkörper (12-12b) als einseitig offener Hohlkörper mit einer sich verjüngenden Gestaltung ausgeführt ist, **dadurch gekennzeichnet, dass** der Hutkörper (12, 12a, 12b) eine schmal zulaufende Spitze aufweist.

2. Medizinische Fluidsteuervorrichtung nach Anspruch 1, **dadurch gekennzeichnet, dass** der Partikelfilter (9-9b) kraft- und/oder form- und/oder stoffschlüssig in dem Anschlussport (3-3b) oder dem Verbindungsport (4-4b, 5-5b) gehalten ist.

3. Medizinische Fluidsteuervorrichtung nach Anspruch 1, **dadurch gekennzeichnet, dass** der Hutkörper (12, 12a, 12b) an einem Stirnendbereich einen Trägerring (10, 10a, 10b) aufweist, der an einer Innenwandung (11, 11a, 11b) des Anschlussports (3, 3a, 3b) oder des Verbindungsports befestigt ist.

4. Medizinische Fluidsteuervorrichtung nach einem der vorhergehenden Ansprüche, **dadurch gekennzeichnet, dass** der Partikelfilter (9, 9a, 9b) in dem Anschlussport (3, 3a, 3b) oder dem Verbindungsport derart befestigt ist, dass die Spitze des Hutkörpers (12, 12a, 12b) distal oder proximal zu einem Anschlussbereich (13, 13a, 13b) des Anschlussports (3, 3a, 3b) oder des Verbindungsports ausgerichtet ist.

5. Medizinische Fluidsteuervorrichtung nach einem der vorhergehenden Ansprüche, **dadurch gekennzeichnet, dass** die Innenwandung des Anschlussports (3-3b) oder des Verbindungsports (4-4b, 5-5b) mit einer Ringprofilierung (11-11b, 11'd) versehen ist, die komplementär auf eine Ringaußenkontur des Trägerrings (10-10b, 10'd) abgestimmt ist.

6. Medizinische Fluidsteuervorrichtung nach Anspruch 5, **dadurch gekennzeichnet, dass** die Ringprofilierung (11-11b, 11'd) als Ringschulter oder als Ringwandung mit einem sich stetig ändernden Durchmesser, insbesondere als konisch gestaltete Ringwandung, ausgeführt ist.

7. Partikelfilter als Teil einer medizinischen Fluidsteuervorrichtung nach einem der vorhergehenden Ansprüche, der die kennzeichnenden Merkmale von wenigstens einem der Ansprüche 1 bis 4 aufweist.

## Claims

1. Medical fluid control device for a medical fluid conduction system, with a fluid flow housing (2 to 2b) which has at least one attachment port (3 to 3b) for attaching and removing an external medical fluid-guiding component and at least one connection port (4 to 4b, 5 to 5b) for connecting to a further functional component of the medical fluid conduction system, and with an adjustment member (8 to 8b) for controlling a flow of fluid relative to the at least one attachment port (3 to 3b) and/or the at least one connection port (4 to 4b, 5 to 5b), wherein a particulate filter (9 to 9b) with a pore width of between 2 µm and 15 µm is integrated in the at least one attachment port (3 to 3b) and/or the at least one connection port (4 to 4b, 5 to 5b), wherein the particulate filter (9 to 9b) is designed as a longitudinally extending hat body (12 to 12b) spatially inside the attachment port (3 to 3b) or the connection port, and wherein the hat body (12 to 12b) is designed as a hollow body open at one end, with a tapering configuration,
**characterized in that**
the hat body (12, 12a, 12b) has a tip narrowing to a point.

2. Medical fluid control device according to claim 1, **characterized in that** the particulate filter (9 to 9b) is held in the attachment port (3 to 3b) or the connection port (4 to 4b, 5 to 5b) with force-fit and/or form-fit engagement and/or by material bonding.

3. Medical fluid control device according to claim 1, **characterized in that** the hat body (12, 12a, 12b) has, at a front end area, a carrier ring (10, 10a, 10b) which is secured on an inner wall (11, 11a, 11b) of the attachment port (3, 3a, 3b) or of the connection port.

4. Medical fluid control device according to one of the preceding claims, **characterized in that** the particulate filter (9, 9a, 9b) is secured in the attachment port (3, 3a, 3b) or the connection port in such a way that the tip of the hat body (12, 12a, 12b) is oriented distally or proximally with respect to an attachment area (13, 13a, 13b) of the attachment port (3, 3a, 3b) or of the connection port.

5. Medical fluid control device according to one of the preceding claims, **characterized in that** the inner wall of the attachment port (3 to 3b) or of the connection port (4 to 4b, 5 to 5b) is provided with an annular profile (11 to 11b, 11'd) which complements an annular outer contour of the carrier ring (10 to 10b, 10'd).

6. Medical fluid control device according to claim 5, **characterized in that** the annular profile (11 to 11b, 11'd) is designed as an annular shoulder or as an annular wall with a constantly changing diameter, in particular as a conically shaped annular wall.

7. Particulate filter as part of a medical fluid control device according to one of the preceding claims, having the characterizing features of at least one of the claims 1 through 4.

## Revendications

1. Dispositif de commande de fluide médical pour un système de conduite de fluide médical, comprenant un boîtier d'écoulement de fluide (2-2b), qui possède au moins un orifice de raccordement (3-3b) pour un raccordement et un retrait d'un composant de guidage de fluide médical externe ainsi qu'au moins un orifice de liaison (4-4b, 5-5b) pour une liaison avec un autre composant fonctionnel du système de conduite de fluide médical, et comprenant aussi un organe de commande (8-8b) destiné à commander un écoulement de fluide par rapport à l'au moins un orifice de raccordement (3-3b) et/ou à l'au moins un orifice de liaison (4-4b, 5-5b), un filtre à particules (9-9b) ayant une taille de pores entre 2 µm et 15 µm étant respectivement intégré dans l'au moins un orifice de raccordement (3-3b) et/ou l'au moins un orifice de liaison (4-4b, 5-5b), le filtre à particules (9-9b) étant configuré sous la forme d'un corps de chapeau (12-12b) étendu en longueur disposé spatialement à l'intérieur de l'orifice de raccordement (3-3b) ou de l'orifice de liaison, et le corps de chapeau (12-12b) étant réalisé sous la forme d'un corps creux ouvert d'un côté avec une configuration qui se rétrécit, **caractérisé en ce que** le corps de chapeau (12, 12a, 12b) possède une pointe effilée fine.

2. Dispositif de commande de fluide médical selon la revendication 1, **caractérisé en ce que** le filtre à particules (9-9b) est maintenu par assemblage de force et/ou par complémentarité de formes et/ou par fusion de matières dans l'orifice de raccordement (3-3b) ou l'orifice de liaison (4-4b, 5-5b).

3. Dispositif de commande de fluide médical selon la revendication 1, **caractérisé en ce que** le corps de chapeau (12, 12a, 12b) possède au niveau d'une zone d'extrémité frontale une bague porteuse (10, 10a, 10b) qui est fixée à une paroi interne (11, 11a, 11b) de l'orifice de raccordement (3, 3a, 3b) ou de l'orifice de liaison.

4. Dispositif de commande de fluide médical selon l'une des revendications précédentes, **caractérisé en ce que** le filtre à particules (9, 9a, 9b) est fixé dans l'orifice de raccordement (3, 3a, 3b) ou l'orifice de liaison de telle sorte que la pointe du corps de chapeau (12, 12a, 12b) est orientée de manière distale ou proximale par rapport à une zone de raccordement (13, 13a, 13b) de l'orifice de raccordement (3, 3a, 3b) ou de l'orifice de liaison.

5. Dispositif de commande de fluide médical selon l'une des revendications précédentes, **caractérisé en ce que** la paroi interne de l'orifice de raccordement (3-3b) ou de l'orifice de liaison (4-4b, 5-5b) est pourvue d'un profilage annulaire (11-11b, 11'd) qui est accordé de manière complémentaire à un contour extérieur annulaire de la bague porteuse (10-10b, 10'd).

6. Dispositif de commande de fluide médical selon la revendication 5, **caractérisé en ce que** le profilage annulaire (11-11b, 11'd) est réalisé sous la forme d'un épaulement annulaire ou d'une paroi annulaire avec un diamètre qui varie continuellement, notamment sous la forme d'une paroi annulaire de configuration conique.

7. Filtre à particules en tant que partie d'un dispositif de commande de fluide médical selon l'une des revendications précédentes, lequel possède les caractéristiques caractérisantes d'au moins l'une des revendications 1 à 4.
